# NEW EUROPEAN PATENT SPECIFICATION

(11) **EP 0 826 766 B2**
(45) Date of publication and mention of the opposition decision: **20.07.2011**
(45) Mention of the grant of the patent: 09.07.2003
(21) Application number: 97306644.2
(22) Date of filing: 29.08.1997
(51) Int. Cl.: C11D 1/10, C11D 1/37, A61Q 19/00, A61Q 19/10, C07C 237/22

(54) **Wash composition**
Waschmittelzusammensetzung
Composition de nettoyage

(30) Priority: 30.08.1996 JP 23069896
(43) Date of publication of application: 04.03.1998
(73) Proprietor: Ajinomoto Co., Inc., Tokyo 104 (JP)
(72) Inventor: Oshimura, Eiko, C. Res. Lab. Ajinomoto Co., Inc., Kawasaki-shi, Kanagawa-ken (JP); Yoshihara, Hideki, C. Res. Lab. Ajinomoto Co. Inc., Kawasaki-shi, Kanagawa-ken (JP); Sakamoto, Kazutami C. Res. Lab. Ajinomoto Co. Inc., Kawasaki-shi, Kanagawa-ken (JP); Hattori, Tatsuya, Tokai Plant, Ajinomoto Co., Inc., Yokkaichi-shi, Mie-ken (JP)
(74) Representative: Nicholls, Kathryn Margaret

(56) References cited:
- EP-A- 0 339 498
- EP-A- 0 827 950
- FR-A- 2 705 673
- JP-A- 106 293
- JP-A- 632 962
- JP-A- 3 111 500
- JP-B- 4 835 058
- US-A- 5 284 602
- TAKEHARA M. ET AL.: 'Surface Activate N-acylglutamate' JAOCS vol. 49, March 1972, pages 157 - 161

## Description

The present invention relates to a wash composition. In particular, it relates to a wash composition comprising an N-long-chain-acyl dipeptide, in which the dipeptide moiety is composed of acidic amino acid residues, and an N-long-chain-acyl acidic amino acid. Embodiments of the wash composition of the present invention may exhibit low irritation to the skin and high resistance to hard water, and give an excellent feeling upon use without any creaking feeling of the hair during rinsing or stretching feeling of the skin after washing.

The expression "creaking feeling" is used herein to describe the way hair sometimes feels to the fingers during rinsing off of detergent. Such a feeling is often very pronounced when hair is washed with soap or with a synthetic detergent for tableware or clothes as such substances tend to remove natural oils from the hair. When hair having a high creaking feeling is rinsed it tends to feel "unlubricated" in the sense that fingers run through the hair during rinsing tend to catch on or in the hair.

Anionic surface active agents such as alkylbenzenesulfonates, higher alcohol sulfates, polyoxyethylene alkyl ether sulfonates and the like have been so far widely used as surface active agents of wash compositions. Wash compositions composed mainly of these anionic surface active agents exhibit an excellent washability, but do not give a satisfactory feeling upon use in view of a creaking feeling in rinsing, a rough feeling after use and the like, and involve big problems such as significant skin irritation and damage to the hair Higher fatty acid-type and phosphate ester-type surface active agents which have a relatively low skin irritation have been hitherto used. These surface active agents have, however, a low resistance to hard water, and are easily bound to calcium in water, whereby a water-insoluble calcium salt (scum) is formed in rinsing. Accordingly, there occur problems such as a decrease in the lathering and a decrease in the lather stability as well as problems of the feeling in use such as the creaking feeling of the hair and the rough feeling of the hair in rinsing.

In recent years, N-acyl acidic amino acid salts which exhibit a low skin irritation, an excellent washability and an excellent feeling upon use have been widely used in wash compositions. The N-long-chain-acyl acidic amino acid salts have been known to be excellent in solution stability inthe weakly acidic pH range which is mild to skin, exhibit less stretching feeling of the skin after use and exhibit less irritation to the skin. On the other hand, as to the N-long-chain-acyl neutral amino acid salts, crystals are liable to separate out in the weakly acidic pH range so that it is difficult to maintain the solution stability, and the lathering ability is remarkably reduced in this weakly acidic range.

The N-long-chain-acyl acidic amino acid salts have been known to have a relatively excellent resistance to hard water, but they are not said to have satisfactory effects. Additionally, they have a problem in that they produce a creaking feeling in the hair when they are used for hair washing. Consequently, the development of a wash composition which retains the benefit of N-long-chain-acyl acidic amino acid salts such as a low skin irritation, and which exhibits an excellent resistance to hard water and less creaking feeling to hair has been in demand.

Meanwhile, N-long-chain-acyl peptides formed by acylating peptides resulting from hydrolysis of natural proteins with higher fatty acids have been also known to be used as a staring material for a wash composition having low skin irritation. These N-long-chain-acyl peptides are used, in many cases, to improve the lathering properties of wash compositions. There is also an example of using the same to improve resistance to hard water. Japanese Laid-Open (Kokai) No. 101,200/1983 discloses that a wash powder containing a higher fatty acid soap and a specific acyl peptide is excellent in scum dispersibility. Further, Japanese Laid-Open (Kokai) No. 65,197/1989 discloses that a wash composition containing a mono-salt of an acylated collagen peptide higher fatty acid is effective for removing metallic ions adhered to the hair because a carboxylic acid side chain of an acidic amino acid residue of the peptide forms a salt with metallic ions.

However, these N-long-chain-acyl peptides are those formed by acylating a mixture of peptides resulting from the hydrolysis of natural proteins. When they are mixed with a liquid wash, the resulting product becomes turbid or has a peculiar odor. Further, solution stability in weakly acidic pH range is not good compared with N-long-chain-acyl acidic amino acid salts and the resistance to hard water is not necessarily satisfactory.

Japanese Laid-Open (Kokai) No. 84,994/1984 proposes N-(N'-long-chain-acylglycyl) glycine salt to conquer the problems such as a turbidity, an odor and the like of products formed by acylating natural proteins, and some wash compositions containing such N-long-chain-acyl neutral amino acid dipeptide salts have been proposed [Japanese Laid-Open (Kokai) Nos. 51,356/1993, 78,693/1993 and 188,694/1995]. With respect to the resistance to hard water of these N-long-chain-acyl neutral amino acid dipeptide salts, Japanese Laid-Open (Kokai) No. 152,999/1984 discloses that the above-mentioned N-(N'-long-chain-acylglycyl)glycine salt exhibits an excellent lathering force in both hard water and soft water. However, the resistance to hard water of N-long-chain acyl neutral amino acid dipeptide salts including N-(N'-long-chain-acylglycyl)glycine salts is not altogether satisfactory, and solution stability in weakly acidic range is not sufficient.

It is an object of the present invention to improve the resistance to hard water and reduce the creaking feeling resulting from use of a wash composition including N-long-chain-acyl acidic amino acid salts. In other words, embodiments of the wash composition of the invention desirably exhibit low irritation and excellent resistance to hard water, are free from the turbidity and odour found in natural peptides, and produce reduced creaking feeling of the hair in rinsing and less stretching sensation of the skin after washing.

Under these circumstances, the present inventors have assiduously conducted investigations, and have consequently found that a wash composition comprising an acylated acidic amino acid dipeptide and an N-long-chain-acyl acidic amino acid may exhibit the above-mentioned properties. This finding has led to the completion of the present invention.

That is, the present invention relates to the use of (A) an N-long-chain-acyl dipeptide represented by formula (1)

R¹-CO-(X-Y)-OM¹ (1)

or a salt thereof,
wherein
X and Y, independently from each other, represent an acidic amino acid residue selected from glutamic acid and aspartic acid,
R¹ represents a linear or branched alkyl or alkenyl group having from 7 to 21 carbon atoms, and
M¹ represents a hydrogen atom, an alkali metal, ammonium, an alkylammonium, an alkanolammonium or a basic amino acid,
in a wash composition also comprising
(B): a N-long-chain-acyl acidic amino acid or its salt, the acidic amino acid being selected from glutamic acid and aspartic acid; and wherein in formula (1), when X is a glutamic acid residue, X may be bound to Y through either an α-carbonyl group or a γ-carbonyl group, and when X is an aspartic acid residue, X may be bound to Y through either an α-carbonyl group or a β-carbonyl group,
for improving the resistance to hard water of the wash composition comprising (B).

In a further aspect, the invention provides a wash composition comprising components (A) and (B) as defined above, wherein the weight ratio of component (A) to component (B) is between 10 : 100 and 20 : 100.

X and Y of the N-long-chain-acyl dipeptide of formula (1) which is Component (A) of the wash composition in the present invention are acidic amino acid residues, and these may be the same or different. The acidic amino acids are selected from glutamic acid and aspartic acid.

When X is a glutamic acid residue, a peptide linkage with Y through a carboxyl group may be provided through either an α-carboxyl group or a γ-carboxyl group. When X is an aspartic acid residue, a peptide linkage with Y through a carboxyl group may be given through either an α-carbonyl group or a β-carbonyl group.

R¹ in the N-long-chain-acyl dipeptide of formula (1) is a linear or branched alkyl or alkenyl group having from 7 to 21 carbon atoms. A linear or branched saturated or unsaturated acyl group having from 7 to 17 carbon atoms is preferable. The long-chain-acyl residue R¹CO which contains the alkyl or alkenyl group can be introduced from fatty acids such as lauric acid, myristic acid, palmitic acid, stearic acid, arachidic acid, coconut oil fatty acid, hardened tallow fatty acid, behenic acid, isostearic acid, oleic acid, castor oil fatty acid, olive oil fatty acid, palm oil fatty acid, and mixtures thereof.

Examples of the salt of the N-long-chain-acyl dipeptide represented by formula (1) include salts of alkali metals such as sodium and potassium; salts of alkanolamines such as triethanolamine; salts of basic amino acids such as lysine and arginine; ammonium salts; and alkylammonium salts. The acidic amino acid residues of X and Y in the N-long-chain-acyl dipeptide of formula (1) have carboxyl groups as side chains, and these carboxyl groups, independently from each other, may form the above-mentioned salts. These salts may be used either singly or in combination.

The N-long-chain-acyl dipeptide of formula (1) or its salt can easily be produced by, for example, a method in which a dipeptide containing an acidic amino acid is formed, and then acylated with a fatty acid halide in an alkaline aqueous solution. The dipeptide can be formed by an ordinary method in the peptide chemistry. Further, it can easily be produced also by a method in which a N-long-chain-acyl amino acid and a carboxyl-protected amino acid are condensed using a condensing agent such as DCC(dicyclohexylcarbodiimide) or the like, and the carboxyl protective group is then selectively removed, or a method in which a N-long-chain-acyl amino acid is then converted to an acid halide using a halogenating agent such as an acid chloride or the like, and this halide is condensed with an amino acid. All combinations of optical activity of the acidic amino acids in the dipeptide are possible - i.e. a combination of L-isomers, a combination of a L-isomer and a D-isomer, a combination of a D-isomer and a L-Disomer or a combination of D-isomers.

Examples of the N-long-chain-acyl dipeptide of formula (1) include N-(N'-long-chain-acyl-α-glutamyl)glutamic acid, N-(N'-long-chain-acyl-γ-glutamyl)glutamic acid, N-(N'-long-chain-acyl-α-aspartyl)aspartic acid, N-(N'-long-chain-acyl-β-aspartyl)aspartic acid, N-(N'-long-chain-acyl-α-glutamyl)aspartic acid, N-(N'-long-chain-acyl-γ-glutamyl)aspartic acid, N-(N'-long-chain-acyl-α-aspartyl)glutamic acid, N-(N'-long-chain-acyl-β-aspartyl)glutamic acid, and salts thereof. These N-long-chain-acyl dipeptides may be either racemic compounds or optically active compounds.

The amino acid residue of the N-long-chain-acyl acidic amino acid as Component (B) of the wash composition in the present invention is derived from glutamic acid, or aspartic acid. The acyl group may be a linear or branched saturated or unsaturated acyl group having from 8 to 22 carbon atoms, preferably from 8 to 18 carbon atoms.

The above-mentioned acyl group can be introduced from fatty acids such as lauric acid, myristic acid, palmitic acid, stearic acid, arachidic acid, coconut oil fatty acid, hardened tallow fatty acid, behenic acid, isostearic acid, oleic acid, castor oil fatty acid, olive oil fatty acid, palm oil fatty acid, and mixtures thereof.

Examples of the salt of the N-long-chain-acyl acidic amino acid include salts of alkali metals such as sodium and potassium; salts of alkanolamines such as triethanolamine; salts of basic amino acids such as lysine and arginine; ammonium salts; and alkylammonium salts. The N-long-chain-acyl acidic amino acids and the salts thereof may be used either singly or in combination.

Examples of the N-long-chain-acyl acidic amino acid include N-lauroyl glutamic acid, N-myristoyl glutamic acid, N-palmitoyl glutamic acid, N-stearoyl glutamic acid, N-oleoyl glutamic acid, N-cocoyl glutamic acid, N-hardened tallow glutamic acid, N-lauroyl aspartic acid, N-myristoyl aspartic acid, N-palmitoyl aspartic acid, N-stearoyl aspartic acid, N-oleoyl aspartic acid, N-cocoyl aspartic acid, N-hardened tallow aspartic acid, and salts thereof. These N-long-chain-acyl acidic amino acids may be used in the form of both an optically active compound and a racemic compound.

The N-long-chain-acyl acidic amino acids and salts thereof can generally be formed by the method using the Schotten Baumann reaction in which an amino acid and a fatty acid halide are condensed in an alkaline aqueous solution, for example, the method described in Japanese Patent Publication Nos. 8,685/1971, 3,058/1973 and 38,681/1976. An amino acid to be acylated may be in the form of a L-isomer, a D-isomer or a racemic compound.

The total amount of the N-long-chain-acyl dipeptide (A) and the N-long-chain-acyl acidic amino acid (B) in the wash composition of the present invention varies depending on the use. In order to provide a satisfactory washability, it is usually 5% by weight or more, preferably between 10 and 80% by weight. However, it may be used in an amount outside this range depending on the use purpose of the wash composition.

The ratio of the N-long-chain-acyl dipeptide (A) to the N-long-chain-acyl acidic amino acid (B) can be set in a relatively wide range. The weight ratio thereof is preferably between 0.1:100 and 20:100, more preferably between 0.5:100 and 10:100. When it is less than 0.1:100. the creaking feeling may become unsatisfactory in some cases. When it exceeds 20:100, the decrease in the feeling upon use is invited in some cases, and it is economically disadvantageous.

The wash composition of the present invention may contain another surface active agent to adjust washability and a lathering properties unless this impairs the effect of the present invention. Examples of other surface active agents include anionic surface active agents such as higher fatty acid salts, alkyl sulfonates, alkylbenzene sulfonates, α-olefin sulfonates, polyoxyethylenealkyl ether sulfonates, N-acyl aminocarboxylates, polyoxyethylenealkyl ether carboxylates, alkyl ether phosphates and sulfosuccinic acids; ampholytic surface active agents such as alkylbetaine surface active agents, amidobetaine surface active agents, alkylsulfobetaine surface active agents, amidosulfobetaine surface active agents and imidazoline surface active agents; nonionic surface active agents such as sugar ether surface active agents, sugar amide surface active agents, sugar ester surface active agents, alkyl saccharide surface active agents, polyoxyethylenealkyl ether surface active agents, higher fatty acid alkanolamide surface active agents and amine oxide surface active agents; and cationic surface active agents such as benzalkonium chloride, a monoalkyl quaternary ammonium salt, a dialkyl quaternary ammonium salt, an N-α-acylarginine lower alkyl ester salt and an N-∈-alkyl lysine lower alkyl ester salt. Further, surface active agents having a low resistance to hard water, such as higher fatty acids or salts thereof, can improve the resistance to hard water by mixing the same with the wash composition of the present invention. That is, it is possible to provide a wash composition comprising a higher fatty acid or its salt as Component (C) in addition to the N-long-chain-acyl dipeptide of formula (1) or its salt as Component (A) and the N-long-chain-acyl acidic amino acid or its salt as Component (B). In this case as well, the amount (weight ratio) of the higher fatty acid or its salt as Component (C) is at most 30% based on the total amount of Components (A), (B) and (C) in order to exhibit well the effect of the resistance to hard water.

The wash composition of the present invention can further contain the other wash starting materials which are commonly used in the wash composition unless impairing the effects of the present invention. Examples thereof include water-soluble high-molecular compounds such as methyl cellulose, hydroxycellulose, hydroxyethyl cellulose and hydroxypropylmethyl cellulose; wetting agents such as propylene glycol, glycerol, 1,3-butylene glycol, polyethylene glycol and sorbitol; viscosity modifiers such as methyl cellulose, ethyl cellulose, hydroxyethyl cellulose, carboxyvinyl polymer, xanthane gum, guar gum, ethanol, polyoxyethylene glycol distearate and polyoxyethylene sorbitan tristearate; hydrocarbons such as liquid paraffin, solid paraffin, vaseline, squalane and olefin oligomer; emulsifiers such as glycerol monoalkyl ester, glycerol monostearate, polyoxyethylenesorbitan monolaurate, polyoxyethylenecetyl ether and polyoxyethylene stearate; higher alcohols such as lauryl alcohol, cetyl alcohol, stearyl alcohol and behenyl alcohol; oils such as a mint oil, an olive oil, a castor oil, a yolk oil, a camellia oil, a soybean oil, a linseed oil, an avocado oil, a jojoba oil and lanolin; ester oils such as isopropyl myristate, isopropyl palmitate, stearyl stearate, octyldodecyl myristate and octyldodecyl oleate; pearling agents such as ethylene glycol distearate and styrene polymer; antiseptics such as methyl paraben and butyl paraben; UV absorbers such as benzophenone derivatives and benzotriazole derivatives; disinfectants such as triclosan; anti-inflammatories such as dipotassium glycyrrhetinate and tocopherol acetate; dandruff preventing agents such as zinc pyrithione; amino acids; drugs; pH adjustors; flavors; pigments; and antioxidants.

In a further aspect, the present invention provides an N-long-chain-acyl dipeptide represented by formula (2) 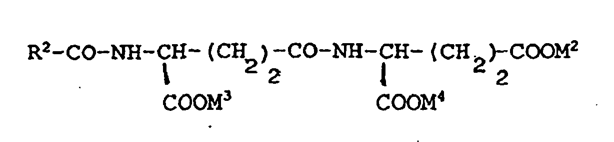 wherein
R² represents a linear or branched alkyl or alkenyl group having from 7 to 21 carbon atoms, and M², M³ and M⁴, independently from each other, represent a hydrogen atom, an alkali metal, ammonium, an alkylammonium, an alkanolammonium or basic amino acid or its salt.

### Examples

The present invention is illustrated more specifically by referring to the following Examples. However, the present invention is not limited thereto.

### Production Example 1

A suspension of 20 g (0.0724 mols) of α-glutamylglutamic acid in 70 ml of water was dissolved in a 27-% sodium hydroxide aqueous solution until the pH reached 11, and 35 ml of acetone were added thereto. To the solution were added dropwise 15.8 g (0.0724 mols) of lauroyl chloride over a period of 1 hour. When adding lauroyl chloride, the temperature was maintained at 10°C, and 27-% sodium hydroxide was added dropwise simultaneously to keep the pH at 11 constantly. After the completion of the addition of lauroyl chloride, the reaction solution was warmed at 30°C, and aged for 30 minutes. Subsequently, the pH was adjusted to 1 with the addition of conc. hydrochloric acid. The solid precipitated was separated through filtration, and dried to obtain 30.6 g of N-(N'-lauroyl-α-glutamyl)glutamic acid in a yield of 92%. This solid was analyzed through infrared spectrophotometry. As a result, a peak of an amide group was observed at 1,650 cm⁻¹ and a peak of a carboxyl group at 1,730 cm⁻¹ respectively.

### Production Example 2

A suspension of 30 g (0.121 mols) of α-aspartylaspartic acid in 115 ml of water was dissolved in a 27-% sodium hydroxide aqueous solution until the pH reached 11, and 58 ml of acetone were added thereto. To the solution were added dropwise 26.9 g (0.121 mols) of cocoyl chloride over a period of 1 hour. When adding cocoyl chloride, the temperature was maintained at 10°C, and 27-% sodium hydroxide was added dropwise simultaneously to keep the pH at 11 constantly. After the completion of the addition of cocoyl chloride, the reaction solution was warmed at 30°C, and aged for 30 minutes. Subsequently, the pH was adjusted to 1 with the addition of conc. hydrochloric acid. The solid precipitated was separated through filtration, and dried to obtain 47.2 g of N-(N'-cocoyl-α-aspartyl)aspartic acid in a yield of 90%. This solid was analyzed through infrared spectrophotometry. As a result, a peak of an amide group was observed at 1,640 cm⁻¹ and a peak of a carboxyl group at 1,730 cm⁻¹ respectively.

### Production Example 3

A suspension of 20 g (0.081 mols) of α-aspartylaspartic acid in 80 ml of water was dissolved in a 27-% sodium hydroxide aqueous solution until the pH reached 11, and 40 ml of acetone were added thereto. To the solution were added dropwise 17.7 g (0.081 mols) of lauroyl chloride over a period of 1 hour. When adding lauroyl chloride, the temperature was maintained at 10°C, and 27-% sodium hydroxide was added dropwise simultaneously to keep the pH at 11 constantly. After the completion of the addition of lauroyl chloride, the reaction solution was warmed at 30°C, and aged for 30 minutes. Subsequently, the pH was adjusted to 1 with the addition of conc. hydrochloric acid. The solid precipitated was separated through filtration, and dried to obtain 31.7 g of N-(N'-lauroyl-α-aspartyl)aspartic acid in a yield of 91%. This solid was analyzed through infrared spectrophotometry. As a result, a peak of an amide group was observed at 1,640 cm⁻¹ and a peak of a carboxyl group at 1,730 cm⁻¹ respectively.

### Production Example 4

A suspension of 16 g (0.058 mols) of γ-glutamylglutamic acid in 55 ml of water was dissolved in a 27-% sodium hydroxide aqueous solution until the pH reached 11, and 27 ml of acetone were added thereto. To the solution were added dropwise 12.6 g (0.058 mols) of lauroyl chloride over a period of 1 hour. When adding lauroyl chloride, the temperature was maintained at 10°C, and 27-% sodium hydroxide was added dropwise simultaneously to keep the pH at 11 constantly. After the completion of the addition of lauroyl chloride, the reaction solution was warmed at 30°C, and aged for 30 minutes. Subsequently, the pH was adjusted to 1 with the addition of conc. hydrochloric acid. The solid precipitated was separated through filtration, and dried to obtain 23.6 g of N-(N'-lauroyl-γ-glutamyl)glutamic acid in a yield of 89%. This solid was analyzed through infrared spectrophotometry. As a result, a peak of an amide group was observed at 1,640 cm⁻¹ and a peak of a carboxyl group at 1,730 cm⁻¹ respectively. FAB mass spectrum: 459(MH⁺)

### Production Example-5

Production Example 1 was repeated using 20.0 g (0.072 mols) of α-glutamylglutamic acid and 21.9 g (0.072 mols)of stearoyl chloride to give 34.9 g of N-(N'-stearoyl-α-glutamyl)glutamic acid in a yield of 89%. This solid was analyzed through infrared spectrophotometry. As a result, a peak of an amide group was observed at 1,650 cm⁻¹ and a peak of a carboxyl group at 1,730 cm⁻¹ respectively.

### Production Example 6

Production Example 1 was repeated using 20.0 g (0.072mols) of α-glutamylglutamic acid and 16.1 g (0.072 mols) of cocoyl chloride to give 30.1 g of N-(N'-cocoyl-α-glutamyl)glutamic acid in a yield of 90%. This solid was analyzed through infrared spectrophotometry. As a result, a peak of an amide group was observed at 1,640 cm⁻¹ and a peak of a carboxyl group at 1,730 cm⁻¹ respectively.

On the following examples, N-long-chain-acyl peptides are used by forming salts with triethanolamine solution, sodium hydride solution or potassium hydride solution. When a triethanolamine salt was formed, pH was adjusted to 5.2. when a sodium salt or potassium salt was formed, pH was adjusted to 5.8. On the test in the following test example 1 to 5, sodium salt is used as N-long-chain acyl peptide salt when N-long-chain-acyl acidic amino acid salt is sodium salt, and triethanolamine salt is used as N-long-chain acylpeptide salt when N-long-chain-acyl acidic amino acid salt is triethanolamine salt.

### Test Example 1

### Test for latheringproperty:

Each wash composition including N-long-chain-acyl acidic amino acid salt(0.5% by weight) and the N-long-chain-acyl dipeptide salt was prepared.

50ml of this solution was stirred for 5 seconds using a domestic mixer ("Millser" trade name for a device of Iwatani International Corporation), and allowed to stand for 1 minute. Then, the amount (ml) of bubbles was measured. City water (calcium concentration 20 ppm) was used to prepare the aqueous solution. When the N-long-chain-acyl acidic amino acid forms a water-insoluble salt with calcium in an aqueous solution, the bubbling property is decreased.

**Table 1**

| Lathering property | | | | | |
|---|---|---|---|---|---|
| | | comparative example | Examples | | |
| Amount of N-(N'-lauroyl-α-glutamyl) glutamic acid salt relative to N-long-chain-acyl acidic amino acid salt | | 0% | 0.5% | 5% | 10% |
| Amount of bubbles (ml) | Triethanolamine | 180 | 187 | 195 | 195 |
| | N-cocoyl glutamate | (193) | | | |
| | Sodium N-cocoyl | 133 | 160 | 175 | 190 |
| | glutamate | (198) | | | |
| | Triethanolamine | 179 | 187 | 197 | 203 |
| | N-lauroyl aspartate | (203) | | | |
| | Sodium N-lauroyl | 173 | 200 | 215 | 238 |
| | aspartate | (253) | | | |
| Values of parentheses are amount of bubbles (ml) using ion-exchanged water(>18MΩ). | | | | | |

**Table 2**

| Lathering property | | | | | |
|---|---|---|---|---|---|
| | | comparative example | Examples | | |
| Amount of N-(N'-lauroyl-γ-glutamyl) glutamic acid salt relative to N-long-chain-acyl acidic amino acid salt | | 0% | 0.5% | 5% | 10% |
| Amount of bubbles (ml) | Triethanolamine | 180 | 185 | 188 | 193 |
| | N-cocoyl glutamate | (193) | | | |
| | Sodium N-cocoyl | 133 | 155 | 195 | 200 |
| | glutamate | (198) | | | |
| | Triethanolamine | 180 | 188 | 195 | 200 |
| | N-lauroyl aspartate | (203) | | | |
| | Sodium N-lauroyl | 173 | 190 | 230 | 235 |
| | aspartate | (253) | | | |
| Values of parentheses are amount of bubbles (ml) using ion-exchanged water (>18MΩ). | | | | | |

**Table 3**

| Lathering property | | | | | |
|---|---|---|---|---|---|
| | | comparative example | Examples | | |
| Amount of N-(N'-lauroyl-α-aspartyl) aspartic acid salt relative to N-long-chain-acyl acidic amino acid salt | | 0% | 0.5% | 5% | 10% |
| Amount of bubbles (ml) | Triethanolamine | 180 | 185 | 190 | 190 |
| | N-cocoyl glutamate | (193) | | | |
| | Sodium N-cocoyl | 133 | 140 | 193 | 205 |
| | glutamate | (198) | | | |
| | triethanolamine N- | 180 | 185 | 195 | 200 |
| | lauroyl aspartate | (203) | | | |
| | Sodium N-lauroyl | 173 | 190 | 223 | 240 |
| | aspartate | (253) | | | |
| Values of parentheses are amount of bubbles (ml) using ion-exchanged water (>18MΩ). | | | | | |

**Table 4**

| Lathering property | | | | | |
|---|---|---|---|---|---|
| | | Comparative examples | | | |
| Amount of coconut oil fatty acid acyl hydrolysis collagen salt relative to N-long-chain-acyl acidic amino acid salt | | 0% | 0.5% | 5% | 10% |
| Amount of bubbles (ml) | Triethanolamine | 185 | 180 | 190 | 180 |
| | N-cocoyl glutamate | (193) | | | |
| | Sodium N-cocoyl | 133 | 140 | 143 | 150 |
| | glutamate | (198) | | | |
| | Triethanolamine | 187 | 140 | 118 | 95 |
| | N-lauroyl aspartate | (203) | | | |
| | Sodium N-lauroyl | 173 | 175 | 167 | 87 |
| | aspartate | (253) | | | |
| Values of parentheses are amount of bubbles (ml) using ion-exchanged water (>18MΩ). | | | | | |

### Test Example 2

### Tests for a creaking feeling:

Five liters of each wash composition (40°C ) including a N-long-chain-acyl acidic amino acid salt (0.5% by weight) and N-long-chain-acyl peptide salt were prepared using water adjusted calcium concentration to 100 ppm. Five panelists washed hair pieces (each 20 g net), and the creaking feeling of the hair was evaluated according to four grades. The results are shown in Table 5 to 7.

**Table 5**

| | | comparative example | Examples | | |
|---|---|---|---|---|---|
| Amount of (N-(N'-lauroyl-α-glutamyl) glutamic acid salt relative to N-long-chain-acyl acidic amino acid salt | | 0% | 0.5% | 5% | 10% |
| Creaking feeling of the hair | Triethanolamine N-cocoyl glutamate | Δ | ○ | ⓞ | ⓞ |
| | Sodium N-cocoyl glutamate | × | Δ | ⓞ | ⓞ |
| | Triethanolamine N-lauroyl aspartate | Δ | ○ | ⓞ | ⓞ |
| | Sodium N-lauroyl aspartate | × | Δ | ⓞ | ⓞ |

| | | | | | |
|---|---|---|---|---|---|
| ⓞ There is no creaking feeling at all. ○ There is little creaking feeling. Δ There is a slight creaking feeling. × There is a creaking feeling. | | | | | |

**Table 6**

| | | comparative example | Examples | | |
|---|---|---|---|---|---|
| Amount of (N-(N'-stearoyl-α-glutamyl) glutamic acid salt relative to N-long-chain-acyl acidic amino acid salt | | 0% | 0.5% | 5% | 10% |
| Creaking feeling of the hair | Triethanolamine N-cocoyl glutamate | Δ | ○ | ⓞ | ⓞ |
| | Sodium N-cocoyl glutamate | × | Δ | ⓞ | ⓞ |
| | Triethanolamine N-lauroyl aspartate | Δ | ○ | ⓞ | ⓞ |
| | Sodium N-lauroyl aspartate | × | Δ | ⓞ | ⓞ |

| | | | | | |
|---|---|---|---|---|---|
| ⓞ There is no creaking feeling at all. ○ There is little creaking feeling. Δ There is a slight creaking feeling. × There is a creaking feeling. | | | | | |

**Table 7**

| | | comparative example | Examples | | |
|---|---|---|---|---|---|
| Amount of (N-(N'-cocoyl-α-aspartyl) aspartic acid salt relative to N-long-chain-acyl acidic amino acid salt | | 0% | 0.5% | 5% | 10% |
| Creaking feeling of the hair | Triethanolamine N-cocoyl glutamate | Δ | ○ | ⓞ | ⓞ |
| | Sodium N-cocoyl glutamate | × | Δ | ⓞ | ⓞ |
| | Triethanolamine N-lauroyl aspartate | Δ | ○ | ⓞ | ⓞ |
| | Sodium N-lauroyl aspartate | × | Δ | ⓞ | ⓞ |

| | | | | | |
|---|---|---|---|---|---|
| ⓞ There is no creaking feeling at all. ○ There is little creaking feeling. Δ There is a slight creaking feeling. × There is a creaking feeling. | | | | | |

### Test Example 3

### Tests for adhesion of scum:

Five liters of each wash composition (40°C ) including a N-long-chain-acyl acidic amino acid salt (0.5% by weight) and N-long-chain-acyl acidic amino acid salt were prepared using water adjusted calcium concentration to 100ppm in a washbowl in which a vinyl chloride black plate was attached to the inner wall surface such that a water level was situated in the center. Five panelists washed hair pieces (each 20 g net), and after the washing, the vinyl chloride plate was taken out, and the amount of scum adhered was evaluated according to five grades. The results are shown in Table 8 to 10.

**Table 8**

| | | comparative example | Examples | | |
|---|---|---|---|---|---|
| Amount of N-(N'-lauroyl-α-glutamyl) glutamic acid relative to N-long-chain-acyl acidic amino acid salt | | 0% | 0.5% | 5% | 10% |
| Amount of scum | Triethanolamine N-cocoyl glutamate | Δ | ○ | ⓞ | ⓞ |
| | Sodium N-cocoyl glutamate | × | Δ | ⓞ | ⓞ |
| | Triethanolamine N-lauroyl aspartate | Δ | ○ | ⓞ | ⓞ |
| | Sodium N-lauroyl aspartate | × | Δ | ⓞ | ⓞ |

| | | | | | |
|---|---|---|---|---|---|
| ⓞ No scum is adhered at all. ○ Scum is little adhered. Δ Scum is slightly adhered. × Scum is adhered. | | | | | |

**Table 9**

| | | Comparative examples | | | |
|---|---|---|---|---|---|
| Amount of N-(N'-lauroyl-glycyl) glycine acid salt relative to N-long-chain-acyl acidic amino acid salt | | 0% | 0.5% | 5% | 10% |
| Amount of scum | Triethanolamine N-cocoyl glutamate | Δ | Δ | Δ | ○ |
| | Sodium N-cocoyl glutamate | × | × | × | Δ |
| | Triethanolamine N-lauroyl aspartate | Δ | Δ | Δ | ○ |
| | Sodium N-lauroyl aspartate | × | × | × | Δ |

| | | | | | |
|---|---|---|---|---|---|
| ⓞ No scum is adhered at all. ○ Scum is little adhered. Δ Scum is slightly adhered. × Scum is adhered. | | | | | |

**Table 10**

| | | Comparative examples | | | |
|---|---|---|---|---|---|
| Amount of coconut oil fatty acid acyl hydrolysis collagen salt relative to N-long-chain-acyl acidic amino acid salt | | 0% | 0.5% | 5% | 10% |
| Amount of scum | Triethanolamine N-cocoyl glutamate | Δ | Δ | Δ | ○ |
| | Sodium N-cocoyl glutamate | × | × | × | Δ |
| | TriethanolamineN-lauroyl aspartate | Δ | Δ | Δ | ○ |
| | Sodium N-lauroyl aspartate | × | × | × | Δ |

| | | | | | |
|---|---|---|---|---|---|
| ⓞ No scum is adhered at all. ○ Scum is little adhered. Δ Scum is slightly adhered. × Scum is adhered. | | | | | |

### Test Example 4

### Odor test:

Each wash composition including N-long-chain-acyl acidic amino acid salt (30% by weight) and the N-long-chain-acyl dipeptide salt was prepared. After these solutions were stored at 40°C for 1 week, the odor was examined. The results are shown in Table 11.

**Table 11**

| | | Examples | | | Comparative examples | | |
|---|---|---|---|---|---|---|---|
| Amount of N-(N'-laurovl-α-glutamyl) glutamic acid relative to N-long-chain-acyl acidic amino acid salt | | 0.5% | 5% | 10% | | | |
| Amount of coconut oil fatty acid acyl hydrolysis collagen based on N-long-chain-acyl acidic amino acid salt | | | | | 0.5% | 5% | 10% |
| Odor | Triethanolamine N-cocoyl glutamate | ○ | ○ | ○ | Δ | Δ | × |
| | Sodium N-cocoyl glutamate | ○ | ○ | ○ | Δ | × | × |
| | Triethanolamine N-lauroyl aspartate | ○ | ○ | ○ | ○ | Δ | × |
| | Sodium N-lauroyl aspartate | ○ | ○ | ○ | Δ | × | × |

| | | | | | | | |
|---|---|---|---|---|---|---|---|
| ○ There is no peculiar odor. Δ There is a slight peculiar odor. × There is a considerable peculiar odor. | | | | | | | |

### Example 5

### Test for turbidity:

Each wash composition including N-long-chain-acyl acidic amino acid salt(30% by weight) and the N-long-chain-acyl dipeptide salt was prepared. After these solutions were stored at 0°C for 1 week, the turbidity was examined. The results are shown in Table 12.

**Table 12**

| | | Examples | | | Comparative examples | | |
|---|---|---|---|---|---|---|---|
| Amount of N-(N'-lauroyl-α-glutamyl)glutamic acid relative to N-long-chain-acyl acidic amino acid salt | | 0.5% | 5% | 10% | | | |
| Amount of coconut oil fatty acid acyl hydrolysis collagen based on N-long-chain-acyl acidic amino acid salt | | | | | 0.5% | 5% | 10% |
| Odor | Triethanolamine N-cocoyl glutamate | ○ | ○ | ○ | ○ | Δ | × |
| | Sodium N-cocoyl glutamate | ○ | ○ | ○ | Δ | × | × |
| | Triethanolamine N-lauroyl aspartate | ○ | ○ | ○ | ○ | Δ | × |
| | Sodium N-lauroyl aspartate | ○ | ○ | ○ | Δ | × | × |

| | | | | | | | |
|---|---|---|---|---|---|---|---|
| ○ There is no turbidity. Δ There is a slight turbidity. × There is a considerable turbidity. | | | | | | | |

### Formulation Example 1

A hair shampoo was prepared using a formulation shown in the following Table 13. This hair shampoo exhibited excellent lathering in hard water, low irritation to the skin and less creaking feeling of the hair in rinsing.

**Table 13**

| (Hair shampoo) | |
|---|---|
| Composition | Content(%) |
| Triethanolamine N- (N'-lauroyl-α-glutamyl) glutamate | 0.5 |
| Triethanolamine N-(N'-lauroyl-γ-glutamyl) glutamate | 0.5 |
| Triethanolamine N-lauroyl glutamate | 18 |
| Triethanolamine lauryl sulfonate | 3 |
| Coconut oil fatty acid dimethylamino acetic acid betaine | 5 |
| Carboxy vinyl polymer | 4 |
| Coconut oil fatty acid diethanolamide | 2 |
| Cationized cellulose | 0.4 |
| Trimethyl glycine | 2 |
| Antiseptic | 0.2 |
| Water | balance |

### Formulation Example 2

A hair shampoo was prepared using a formulation shown in the following Table 14. This hair shampoo exhibited an excellent lathering in hard water, low irritation to the skin and less creaking feeling of the hair in rinsing.

**Table 14**

| (Hair shampoo) | |
|---|---|
| Composition | Content(%) |
| Triethanolamine N-(N'-cocoyl-α-aspartyl) aspartate | 2 |
| Triethanolamine N-cocoyl aspartate | 20 |
| Coconut oil fatty acid diethanolamide | 4 |
| Cationized cellulose | 0.4 |
| POE(60)polymyristylene(1)tallow alkyl ether | 2.5 |
| Hydrolyzed collagen | 0.5 |
| Distearyl polyethlene glycol | 2 |
| Glycerol | 5 |
| Antiseptic | 0.2 |
| perfume | 0.1 |
| Water | balance |

### Formulation Example 3

A cleansing cream was prepared using a formulation shown in the following Table 15. This cleansing cream exhibited an excellent in bubbling property in hard water, exhibited excellent lathering in hard water, low irritation to skin and less stretching feeling of the skin after washing.

**Table 15**

| (Cleansing cream) | |
|---|---|
| Composition | Content(%) |
| Sodium N-(N'-cocoyl-α-glutamyl) glutamate | 24 |
| Arginine N-cocoyl glutamate | 10 |
| Sodium stearate | 1 |
| Coconut oil fatty acid diethanolamide | 4 |
| Distearyl polyethlene glycol | 2 |
| Sorbitol | 2 |
| Hydroxymetyl cellulose | 0.8 |
| POE(120)metyl glucose dioleate | 0.5 |
| propylene glycol | 10 |
| Antiseptic | 0.2 |
| perfume | 0.1 |
| Water | balance |

### Formulation Example 4

A body shampoo was prepared using a formulation shown in the following Table 16. This cleansing cream exhibited an excellent lathering in hard water, low irritation to the skin and less stretching feeling of the skin after washing.

**Table 16**

| (Body shampoo) | |
|---|---|
| Composition | Content(%) |
| Potassium N-(N'-cocoyl-α-glutamyl) glutamate | 6 |
| Arginine N-cocoyl glutamate | 15 |
| Coconut oil fatty acid potassium salt | 4 |
| Coconut oil fatty acid diethanolamide | 3 |
| Cationized guar gum | 1 |
| Butylene glycol | 3 |
| citric acid mono hydrate | suitable amount |
| Antiseptic | 0.2 |
| perfume | 0.1 |
| Water | balance |

Embodiments of the wash composition of the present invention may exhibit low irritation and an excellent resistance to hard water, be free from turbidity and odour observed in natural peptides, and exhibit less creaking feeling of the hair and less stretching feeling of the skin.

## Claims

1. Use of (A)an N-long-chain-acyl dipeptide represented by formula (1)
R¹-CO-(X-Y)-OM¹ (1)
or a salt thereof
wherein
X and Y, independently from each other, represent an acidic amino acid residue selected from glutamic acid and aspartic acid,
R¹ represents a linear or branched alkyl or alkenyl group having from 7 to 21 carbon atoms, and
M¹ represents a hydrogen atom, an alkali metal, ammonium, an alkylammonium, an alkanolammonium or a basic amino acid, in a wash composition also comprising (B):a N-long-chain-acyl acidic amino acid or its salt, the acidic amino acid being selected from glutamic acid and aspartic acid: and wherein in formula (1), when X is a glutamic acid residue, X may be bound to Y through either an α-carbonyl group or a γ-carbonyl group, and when X is an aspartic acid residue, X may be bound to Y through either anα-carbonyl group or a β-carbonyl group,
for improving the resistance to hard water of the wash composition comprising (B).

2. The use of claim 1 wherein the weight ratio of Component (A) to Component (B) is between 0.1: 100 and 20:100.

3. A wash composition comprising: (A)an N-tongchain-acyl dipeptide represented by formula (1)
R¹-CO-(X-Y)-OM¹ (1)
or a salt thereof
wherein
X and Y, independently from each other, represent an acidic amino acid residue selected from glutamic acid and aspartic acid,
R¹ represents a linear or branched alkyl or alkenyl group having from 7 to 21 carbon atoms, and
M¹ represents a hydrogen atom, an alkali metal, ammonium, an alkylammonium, an alkanolammonium or a basic amino acid;
and (B) a N-long-chain-acyl acidic amino acid or its salt, the acidic amino acid being selected from glutamic acid and aspartic acid; and wherein in formula (1), when X is a glutamic acid residue, X may be bound to Y through either an α-carbonyl group or a γ-carbonyl group, and when X is an aspartic acid residue, X may be bound to Y through either anα-carbonyl group or a β-carbonyl group;
wherein the weight ratio of Component (A) to Component (B) is between 10:100 and 20:100.

4. A N-long-chain-acyl dipeptide represented by formula (2) 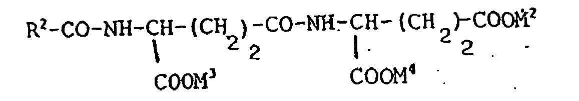 wherein
R² represents a linear or branched alkyl or alkenyl group having from 7 to 21 carbon atoms, and
M², M³ and M⁴, independently from each other, represent a hydrogen atom, an alkali metal, ammonium, an alkylammonium, an alkanolammonium or a basic amino acid
or its salt.

## Patentansprüche

1. Verwendung (A) eines N-Langkettenacyldipeptids der Formel (1)
R¹-CO-(X-Y)-OM¹ (1)
oder eines Salzes davon,
worin
X und Y unabhängig voneinander den Rest einer sauren Aminosäure, ausgewählt unter Glutaminsäure und Asparaginsäure, darstellen, R¹ eine lineare oder verzweigte Akyl- oder Alkenylgruppe mit 7 bis 21 Kohlenstoffatomen darstellt und M¹ ein Wasserstoffatom, ein Alkalimetall, Ammonium, ein Alkylammonium, ein Alkanolammonium oder eine basische Aminosäure darstellt,
in einer Waschmittelzusammensetzung, die ebenfalls (B) enthält:
eine saure N-Langkettenacylaminosäure oder ihr Salz,
wobei die saure Aminosäure unter Glutaminsäure und Asparaginsäure ausgewählt ist;
worin für Formel (1) gilt, daß wenn X ein Glutaminsäurerest ist, entweder über eine α-Carbonylgruppe oder eine γ-Carbonylgruppe an Y gebunden sein, und wenn X ein Asparaginsäurerest ist, X entweder über eine α-Carbonylgruppe oder eine β -Carbonylgruppe an Y gebunden sein kann,
zur Verbesserung der Beständigkeit der (B) enthaltenden Waschmittelzusammensetzung gegenüber hartem Wasser.

2. Verwendung nach Anspruch 1, worin das Gewichtsverhältnis der Komponente (A) zur Komponente (B) zwischen 0,1:100 und 20:100 ist.

3. Waschmittelzusammensetzung: (A) ein N-Langkettenacyldipeptid der Formel (1)
R¹-CO-(X-Y)-OM¹ (1)
oder ein Salz davon,
worin
X und Y unabhängig voneinander den Rest einer sauren Aminosäure, ausgewählt unter Glutaminsäure und Asparaginsäure, darstellen, R¹ eine lineare oder verzweigte Alkyl- oder Alkenylgruppe mit 7 bis 21 Kohlenstoffatomen darstellt und M¹ ein Wasserstoffatom, ein Alkalimetall, Ammonium, ein Alkylammonium, ein Alkanolammonium oder eine basische Aminosäure darstellt,
und (B) eine saure N-Langkettenacylaminosäure oder ihr Salz,
wobei die saure Aminosäure unter Glutaminsäure und Asparaginsäure ausgewählt ist,
worin für Formel (1) gilt, daß wenn X ein Glutaminsäurerest ist, entweder über eine α-Carbonylgruppe oder eine γ-Carbonylgruppe an Y gebunden sein, und wenn X ein Asparaginsäurerest ist, X entweder über eine α-Carbonylgruppe oder eine β -Carbonylgruppe an Y gebunden sein kann,
worin das Gewichtsverhältnis der Komponente (A) zur Komponente (B) zwischen 10:100 und 20:100 ist.

4. N-Langkettenacyldipeptid der Formel (2) 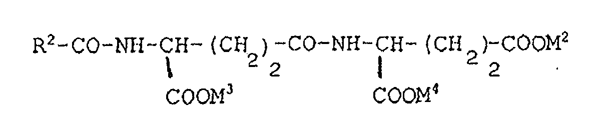 worin R² eine lineare oder verzweigte Alkyl- oder Alkenylgruppe mit 7 bis 21 Kohlenstoffatomen darstellt und M², M³ und M⁴ unabhängig voneinander ein Wasserstoffatom, ein Alkalimetall, Ammonium, ein Alkylammonium, ein Alkanolammonium oder eine basische Aminosäure oder deren Salz darstellen.

## Revendications

1. Utilisation de (A) un dipeptide à chaîne N-acyle longue représenté par la formule (1)
R¹-CO-(X-Y)-OM¹ (1)
ou un sel de celui-ci,
dans laquelle
X et Y, indépendamment l'un de l'autre, représentent un reste acide d'acide aminé choisi parmi l'acide glutamique et l'acide aspartique,
R¹ représente un groupe alkyle ou alcényle linéaire ou ramifié ayant de 7 à 21 atomes de carbone, et
M¹ représente un atome d'hydrogène, un métal alcalin, un groupe ammonium, alkylammonium, alcanolammonium ou acide aminé basique, dans une composition de lavage comprenant également
(B) : un acide aminé N-acyle-acide à chaîne longue ou son sel, l'acide aminé acide étant choisi parmi l'acide glutamique et l'acide aspartique ; et dans laquelle dans la formule (1), quand X est un reste acide glutamique, X peut être lié à Y par un groupe α-carbonyle ou un groupe γ-carbonyle, et quand X est un reste acide aspartique, X peut être lié à Y par un groupe α-carbonyle ou un groupe β-carbonyle,
pour améliorer la résistance à l'eau dure de la composition de lavage comprenant (B).

2. Utilisation selon la revendication 1, dans laquelle le rapport en poids du composant (A) au composant (B) est de 0,1 : 100 à 20 : 100.

3. Composition de lavage comprenant : (A) un dipeptide à chaîne N-acyle longue représenté par la formule (1) :
R¹-CO-(X-Y)-OM¹ (1)
ou un sel de celui-ci,
dans laquelle
X et Y, indépendamment l'un de l'autre, représentent un reste acide d'acide aminé choisi parmi l'acide glutamique et l'acide aspartique,
R¹ représente un groupe alkyle ou alcényle linéaire ou ramifié ayant de 7 à 21 atomes de carbone, et
M¹ représente un atome d'hydrogène, un métal alcalin, un groupe ammonium, alkylammonium, alcanolammonium ou acide aminé basique ;
et (B) un acide aminé N-acyle-acide à chaîne longue ou son sel, l'acide aminé acide étant choisi parmi l'acide glutamique et l'acide aspartique ; et dans laquelle dans la formule (1), quand X est un reste acide glutamique, X peut être lié à Y par un groupe α-carbonyle ou un groupe γ-carbonyle, et quand X est un reste acide aspartique, X peut être lié à Y par un groupe α-carbonyle ou un groupe β-carbonyle ;
dans laquelle le rapport en poids du composant (A) au composant (B) est de 10 : 100 à 20 : 100.

4. Dipeptide à chaîne N-acyle longue représenté par la formule (2) 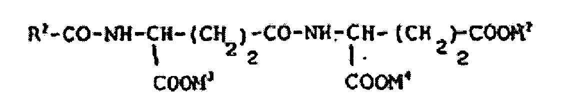 dans laquelle
R² représente un groupe alkyle ou alcényle linéaire ou ramifié ayant de 7 à 21 atomes de carbone, et
M², M³ et M⁴, indépendamment l'un de l'autre, représentent un atome d'hydrogène, un métal alcalin, un groupe ammonium, alkylammonium, alcanolammonium ou acide aminé basique ou son sel.
